# EUROPEAN PATENT SPECIFICATION

(11) **EP 2 705 816 B1**
(45) Date of publication and mention of the grant of the patent: **16.01.2019**
(21) Application number: 13183595.1
(22) Date of filing: 09.09.2013
(51) Int. Cl.: A61F 15/00

(54) **Device for applying a bandage around a limb**
Vorrichtung zum Anlegen eines Verbands um ein Glied
Dispositif pour application d'un bandage autour d'un membre

(30) Priority: 07.09.2012 EP 12183530
(43) Date of publication of application: 12.03.2014
(73) Proprietor: Dona + Beheer B.V., 5504 KA Veldhoven (NL); Settels Savenije group of companies B.V., 5651 GW Eindhoven (NL); P&H Adviseurs Bouw-en Vastgoed B.V., 5504 KA Veldhoven (NL)
(72) Inventor: Dieleman, Maria, 6417 GR Heerlen (NL); van Rens, Piet Christiaan Jozef, 5721 LE Asten (NL); Dona, Marinus Josephus Jacobus, 5504 KA Veldhoven (NL); Peeters, Eugène Adrianus Franciscus Maria, 5504 KA Veldhoven (NL)
(74) Representative: Dekker-Garms, Alwine Emilie

(56) References cited:
- EP-A1- 2 485 227
- DE-U1-202005 006 876
- GB-A- 1 205 162
- GB-A- 2 184 026
- NL-C- 1 037 955
- US-A- 5 238 202
- US-A- 5 421 534
- US-A1- 2010 243 786
- US-B1- 6 508 430
- ANDREW LAWLESS: "Adpal offer high speed wrapping machine", INTERNET CITATION, 13 August 2009 (2009-08-13), pages 1-2, XP002608232, Retrieved from the Internet: URL:http://warehousenews.co.uk/?p=4788 [retrieved on 2010-11-04]

## Description

The present invention relates to a device which is suitable to be used for the purpose of a process of applying a bandage around a limb, and which comprises a rotatable support for supporting a bandage roll; and an interface for receiving input information representing a desired tension of the bandage during a bandage applying process.

For various medical reasons, it may be necessary to apply a bandage around a limb of a person or an animal in order to exert a certain pressure on the limb. For example, when a person is suffering from fluid-filled areas in the leg, a doctor may determine that the swollen leg is subjected to compression bandaging every day or every other day, so that it is eventually possible to use an elastic support stocking on the leg and to further treat the leg by means of the stocking. It is very difficult for medical staff members to perform the process of applying a bandage in such a way that a predetermined pressure is actually realized, as it is only possible for them to rely on feeling based on experience. As a result, in many practical cases, it appears that the pressure is too high or too low. Hence, there is a need for a device which is suitable to be used for the purpose of a process of applying a bandage around a limb, which device will hereinafter be referred to as bandage applicator, and which can assist the medical staff members with unwinding a bandage roll and wrapping the bandage around a limb in such a way that a correct pressure is obtained.

Various types of bandage applicators are known in the art. For example, WO 2011/025813 discloses a bandage applicator comprising a handle to be held by a user of the applicator, wherein the handle is coupled to a support in the form of a spindle. Furthermore, the applicator comprises a brake mechanism which is integral with the handle for controlling a tension of the bandage as it is applied to a limb. The bandage may be pre-wound on a spool that rotates around the spindle. Actuation of the braking mechanism increases pressure on the rotating spool in order to increase tension on the bandage. Alternatively, the spindle rotates in conjunction with a shaft within the handle. In that case, the braking mechanism applies pressure against the rotating shaft to increase tension in the bandage. Pre-wound cartridges of rolled bandage material may be successively loaded to the applicator's spindle for repeated usage of the applicator.

US 5,065,865 discloses a holder for a bandage and a tension roller journalled in the holder. The roller is controlled so that the force required to roll it is adjustable. The bandage is entrained over the roller and the surface of the roller is formed so that there is positive engagement of the bandage with the roller whereby the tension in the bandage is controlled. The holder includes an enclosure formed by end walls and curved piece, which serves to hold a bandage roll. From the enclosure, the bandage runs under an extension, thence over a drum or roller having an axle journalled in the side walls in front of the enclosure. This axle may take the form of a long bolt having a head at one end and a thumb nut threaded onto the bolt at the other. Bushings on the axle and between the ends of the roller and the side walls of the enclosure hold the rollers properly spaced, and provide braking for the roller as the thumb nut is tightened to press the side walls against the bushings. In order to have a possibility of replicating the pressure, a scale may be provided on the side wall under the nut. By noting the position of the nut on the scale when proper pressure is applied, and then using a similar setting of the nut, a duplicate -or near duplicate- of the pressure should be achieved.

GB 2 333 506 discloses a bandage applicator which comprises a support in the form of an axle around which a bandage having an adhesive element is wound. The axle is rotated within a handle, and a tension regulator is provided to control the degree of friction between the handle and the axle as the bandage is unwound from the applicator.

GB 2 369 815 discloses a bandage applicator which comprises a cylindrical body rotatable within a housing which forms a handle, and means at one end of the cylindrical body for mounting a bandage roll. The housing may be provided by two opposing half-casings, which are preferably cylindrical and concentric with the cylindrical body. The half-casings attach around a projection located on the cylindrical body, and further comprise interlocking studs and opposing grooves. The cylindrical body may also have a cylindrical extension fitted with longitudinal slots for engaging longitudinal ridges on a tubular body. The cylindrical body may have a projection in a region adjacent the handle, which, when pressurized by a user's finger, brakes the rotation of the cylindrical body within the housing in order to regulate the tension of the bandaging. The projection may further comprise linear ridges to facilitate the grip of the user's finger. The bandage material may be rolled around the tubular body, attached via an adhesive element. The bandage material may also have an adhesive element to allow the beginning of the bandage to be found.

US 2010/243786 discloses a winding apparatus for and a method of applying a winding of a band-like material to a limb of a transformer core. A common control device is used for controlling both a servomotor for displacing a carriage on which a rotary member is present and a motor for rotating the rotary member. A pin for receiving a roll carrying a band-like material is braked such that an appropriate tension occurs in the band-like material during application of the band-like material to the core limb.

It appears from the description of the various documents mentioned in the foregoing that it is known in the art to have a bandage applicator in which regulating the tension of the bandage involves applying pressure in order to have a braking action directly on the bandage, or on the support on which a bandage roll is mounted.

It is an objective of the present invention to provide another type of bandage applicator, particularly a bandage applicator in which the tension prevailing inside a bandage to be applied to a limb is set according to another principle than the known principle of performing a braking action in a mechanical manner, i.e. by having friction between at least two elements which are moving with respect to each other.

The objective of the present invention is achieved by means of a device which is suitable to be used for the purpose of a process of applying a bandage around a limb, which is a handheld device having a grip for allowing a user of the device to take hold of the device, and which comprises a rotatable support for supporting a bandage roll; a motor for driving the support; a bidirectional drive circuit for driving the motor; an interface for receiving first input information representing a desired tension of the bandage during a bandage applying process; a detecting mechanism for receiving second input information representing an actual tension of the bandage; and a controller for controlling operation of the motor during a bandage applying process through the drive circuit, which is connected to both the interface for receiving the first input information from the interface and the detecting mechanism for receiving the second input information from the detecting mechanism, which is configured to determine a value representing an output torque of the motor on the basis of the first and second input information, and which is connected to the drive circuit for setting the value in the drive circuit.

According to the present invention, controlling the tension in a bandage in a process of wrapping the bandage around a limb involves controlling an output torque of the motor which is connected to the support, on the basis of both information in respect of a desired tension of the bandage and information in respect of an actual tension of the bandage. Realizing a correct tension is done in a most accurate way by setting an output torque instead of performing a braking action as known in the art. During operation of the bandage applicator according to the present invention, the motor acts to retract the bandage as long as a user of the bandage applicator exerts a pulling force on the bandage in a process of wrapping the bandage around a limb by means of the applicator. In case the user stops pulling at the bandage, for example, when putting the bandage applicator from one hand into another, the bandage tends to wind around the support again, as a result of which sagging of the bandage and loss of tension in the bandage are prevented. By controlling the output torque of the motor, wherein both magnitude and direction of the output torque are determined and set, it is guaranteed that the correct tension is always prevailing in the bandage.

The bandage applicator according to the present invention is very easy to use. In fact, the bandage applicator is realized as a handheld device, so that a user of the bandage applicator does not need to work in another way as compared to a situation in which a bandage is applied around a limb by hand, i.e. without using a device. However, a major difference with such a situation is that the user is no longer required to only rely on feeling when it comes to exerting a pulling force on the bandage while applying the bandage. According to the present invention, the user can simply pull at the bandage with a force which is sufficient for unwinding the bandage from the support, wherein the exact tension in the bandage is obtained automatically by appropriate control of the motor, particularly the output torque of the motor, which results in a certain pulling force through the bandage roll.

The exact value of the output torque is determined on the basis of two types of input information, namely input information representing a desired tension of the bandage and input information representing an actual tension of the bandage, wherein the latter type of input information is used for making corrections to the output torque of the motor in case the actual tension appears to deviate from the desired tension. When, at a certain moment during a bandage applying process, it is desired to have a higher tension of the bandage, the output torque is increased. The first input information can be varied over time during a bandage applying process if so desired, for example, depending on the position of the bandage on a limb, wherein the output torque of the motor is varied in a similar manner.

Assuming that the bandage applicator according to the present invention further comprises a support frame for supporting various components of the bandage applicator, it is a feasible possibility for the detecting mechanism to comprise both means which are movable with respect to the support frame under the influence of an actual pulling force prevailing in the bandage, and means for detecting an actual position of the movable means with respect to the support frame. For example, the means which are movably arranged may comprise a motor housing for accommodating the motor, i.e. a component which is directly under the influence of the reaction to the action of the motor. In particular, the motor housing may be rotatably arranged on the support frame. In that case, according to one practical possibility, the detecting means may comprise a combination of a projecting element and an optical sensor for detecting the projecting element, wherein the projecting element is arranged on one of the support frame and the motor housing, wherein the optical sensor is arranged on another of the support frame and the motor housing, and wherein the controller is connected to the optical sensor. Furthermore, in that case, it is possible for the bandage applicator to comprise resilient means such as a leaf spring or a coil spring for connecting the motor housing to the support frame and thereby delimiting movement of the motor housing with respect to the support frame.

In a practical embodiment of the bandage applicator according to the present invention, the motor is an electromotor, wherein the controller is configured to determine a value representing a supply of current to the motor on the basis of the first and second input information, and to control the drive circuit for realizing the value. Hence, the working principle of this embodiment is based on the fact that the output torque of an electromotor is determined by the current that is supplied to the motor, wherein the current may be set. In this embodiment, the controller may be configured to determine a voltage to be set in the drive circuit. Setting a current on the basis of an input voltage is possible when the drive circuit is adapted to realize a direct relation between the voltage and the current. For example, this is the case when the drive circuit comprises a metal-oxide-semiconductor field-effect transistor (MOSFET), an operational amplifier and a resistor, wherein the operational amplifier is connected to the controller for receiving an incoming voltage set by the controller, and wherein the operational amplifier is arranged for letting a voltage across the resistor be the same as the incoming voltage and thereby determining a value of a current in the drive circuit and determining an opened/closed state of the MOSFET.

For realizing a predetermined tension in a bandage, the output torque of the motor is set at an appropriate value by setting the current to be supplied to the motor. There is a direction relation between the current, the output torque and the tension of the bandage, wherein the diameter of the bandage roll constitutes a factor in the relation between the latter two values. By using a circuit as mentioned, in which a MOSFET, an operational amplifier and a resistor are arranged besides the motor and a power source, it is possible to link the value of the current directly to a value of an input voltage. The MOSFET is a type of transistor, which can be used as an electrically controlled switch. In this case, the operational amplifier is used for determining the opened/closed state of the MOSFET. The operational amplifier is an electrical component which can be used as a voltage follower, wherein a voltage drop across the resistor equals the input voltage at the operational amplifier. In this way, the input voltage can be used to directly determine the current to be supplied to the motor.

In respect of the power source, it is noted that it is advantageous to use a power source in the form of a battery. In such a case, optimal freedom of movement of the bandage applicator is obtained, which can be desirable in the context of bandage applying processes.

In general, as mentioned earlier, the bandage applicator according to the present invention is a handheld device having a grip for allowing a user of the applicator to take hold of the device. The interface which is used for transferring input information to the controller of the bandage applicator can comprise a potentiometer, wherein a user can set the correct tension for a bandage by simply rotating a knob, for example.

The bandage applicator according to the present invention offers a possibility of receiving input information in an automated manner. In particular, the interface may be adapted to communicate with a computer through the Internet. Additionally or alternatively, the interface may be adapted to read information from a digital card.

On the other hand, it is possible for the bandage applicator to provide information about bandage applying processes which have been performed by means of the applicator. To that end, the bandage applicator may comprise a memory for storing information regarding the voltage in the drive circuit for driving the motor, which can be used as a representative of the tension in the bandage as explained in the foregoing. In general, the memory may be adapted to store information regarding the value representing the output torque of he motor as set in the drive circuit. In a practical embodiment, the bandage applicator further comprises a transmitter for transmitting information from the memory to a location outside of the applicator. In order to preserve a wireless appearance of the bandage applicator, the transmitter may be adapted to transmit information in a wireless fashion.

According to another option, the bandage applicator may be equipped with an encoder for registering length and position of applied bandages, wherein the encoder is located at an output shaft of the motor. Also, it is possible to provide a measuring device which is suitable to be put on the bandage applicator in the place of a bandage, i.e. on the support, and which is adapted to measuring dimensions of limbs, particularly a thickness of limbs. By performing measurements of the thickness of limbs, it is possible to check whether improvements are made by using the bandages and applying a certain pressure to the limbs by means of the bandages, and to which extent the thickness is decreased.

All in all, various ways of providing information to the bandage applicator and obtaining information from the bandage applicator are possible within the framework of the present invention. When the Internet is used, for example, it is possible for a doctor to monitor the bandage applying processes which are performed on a patient, and also to provide appropriate information for future processes. Especially when the electromotor as described in the foregoing and the electrical circuit for controlling the current to be supplied to the motor are used, it is relatively simple to equip the bandage applicator with suitable components for realizing exchange of information between the applicator and the external world.

In a further automated embodiment, the bandage applicator according to the present invention may comprise a scanner for reading information on a bandage roll representing the type of the bandage roll, wherein the scanner is connected to the controller. Various types of bandage rolls may be provided with various barcodes, and the scanner may be a barcode reader. It may be relevant to have information about the type of bandage roll, especially in view of the fact that rolls of self-adhesive bandage exist, which require a higher torque for unwinding.

The above-described and other aspects of the present invention will be apparent from and elucidated with reference to the following detailed description of two embodiments of a handheld bandage applicator which is adapted to set a required tension in a bandage during a bandage applying process by controlling an output torque of a motor which is present in the applicator for driving a support for supporting a bandage roll.

The present invention will now be explained in greater detail with reference to the figures, in which equal or similar parts are indicated by the same reference signs, and in which:
figure 1 shows a perspective view of a first embodiment of the bandage applicator according to the present invention and a bandage roll arranged on the bandage applicator;
figure 2 shows a sectional view of the first embodiment of the bandage applicator and the bandage roll arranged on the bandage applicator; and
figure 3 is an electrical diagram of a controller of the first embodiment of the bandage applicator and a drive circuit for driving the motor of the bandage applicator;
figure 4 shows a perspective view of a second embodiment of the bandage applicator according to the present invention and a bandage roll arranged on the bandage applicator;
figure 5 shows a sectional view of the second embodiment of the bandage applicator and the bandage roll arranged on the bandage applicator;
figure 6 illustrates a mutual positioning of a number of components of the second embodiment of the bandage applicator and the bandage roll arranged on the bandage applicator, wherein the components shown include a motor for driving a rotatable support for supporting the bandage roll and a rotatably arranged motor housing for accommodating the motor, and wherein a direction in which the bandage is to be wound off the bandage roll is indicated by means of an arrow; and
figure 7 illustrates three possible positions of the motor housing in the second embodiment of the bandage applicator.

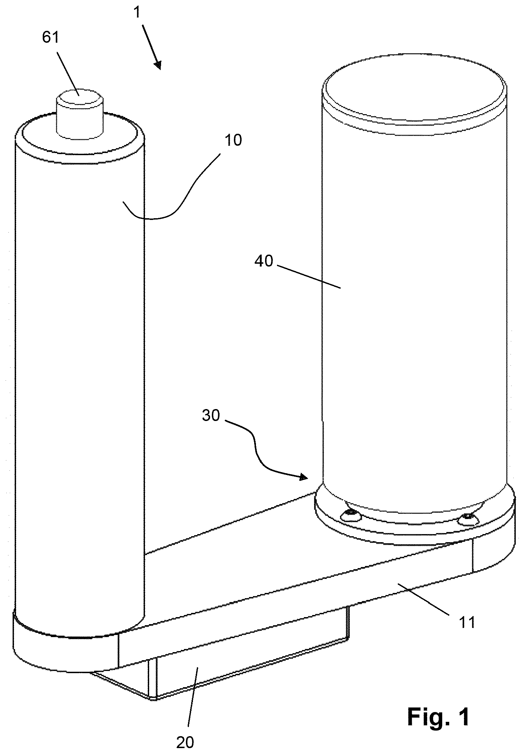

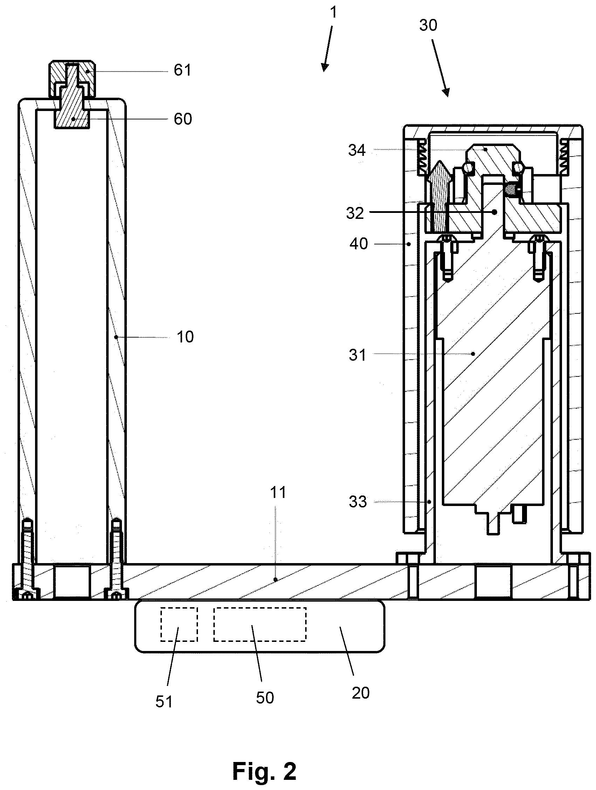

Figure 1 shows a perspective view of a first embodiment of the bandage applicator 1 according to the present invention, which will hereinafter be referred to as first bandage applicator 1, and figure 2 shows a sectional view of the first bandage applicator 1. The first bandage applicator 1 is provided in the form of a handheld device, and comprises a grip 10 for allowing a user of the device to take hold of the device. The grip 10 is supported on a plate 11 which serves to support two other main components of the first bandage applicator 1 besides the grip 10, namely an electrical box 20 for accommodating a battery and various other electrical components as will be explained later on, and an assembly 30 for supporting and driving a bandage roll 40.

Figure 2 shows that the assembly 30 for supporting and driving a bandage roll 40 comprises an electromotor 31 having an output shaft 32, which is arranged in a housing 33, and further comprises a support 34 for supporting a bandage roll 40, wherein the support 34 is arranged on the output shaft 32 of the motor 31. Hence, the support 34 is driven by the motor 31 during operation of the first bandage applicator 1. When it is intended to use the first bandage applicator 1 in a process of applying a bandage around a limb such as a leg, a bandage roll 40 is provided and coupled to the support 34. The support 34 can be equipped with any suitable means for establishing a coupling which is both releasable and reliable.

The first bandage applicator 1 offers a possibility for handling a bandage roll 40 and unwinding the bandage in a convenient manner, as a user of the applicator 1 can hold the grip 10 during a bandage applying process, and continuously move the applicator 1 such as to encircle to limb to be enwrapped in the bandage. Moreover, the first bandage applicator 1 offers a possibility for realizing a correct tension in the bandage during the bandage applying process, as the applicator 1 is adapted to accurately control the operation of the motor 31 as will be explained in the following.

A principle underlying the design of the first bandage applicator 1 is that the tension prevailing in a bandage is not controlled by braking the bandage, but by using the motor 31 for realizing a certain torque at the support 34. By using a motor 31 and controlling the output torque of the motor 31, the tension in the bandage is accurately set, independent of the extent to which a user of the first bandage applicator 1 pulls at the bandage by means of the applicator 1. In fact, the pulling action taken by a user causes the bandage to unwind from the roll 40 without influencing the tension prevailing in the bandage. If, during a bandage applying process, a user starts to pull harder at the bandage, the speed at which the bandage is unwound from the roll 40 is increased, while the tension in the bandage is not changed. If, during a bandage process, a user releases the bandage, the bandage is wound back on the roll 40 again until the user restores the pulling action.

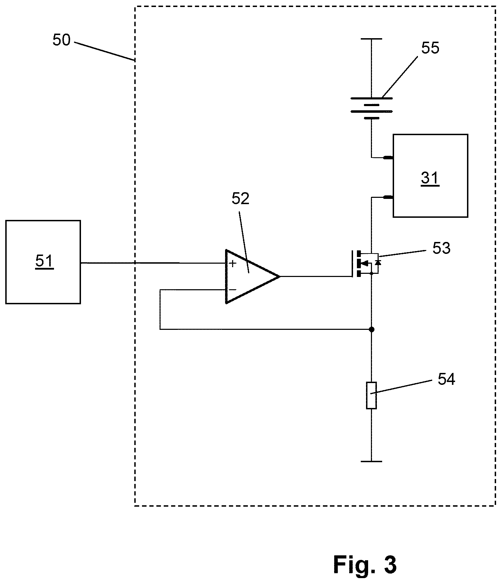

In general, for the purpose of controlling the operation of the motor 30 so that a desired tension is realized in a bandage, the first bandage applicator 1 comprises a drive circuit for driving the motor 31, an interface for receiving input information representing the desired tension, and a controller which is connected to both the interface and the drive circuit. In the shown example, the drive circuit is a circuit 50 comprising electrical components as diagrammatically shown in figure 3, which are located inside the electrical box 20. The controller 51 can be a programmable chip, for example, and may be located inside the electrical box 20 as well. In figure 2, both the controller 51 and the drive circuit 50 are indicated by means of dashed boxes. In figure 3, the controller 51 is diagrammatically depicted in the form of a box which is connected to the drive circuit 50. Furthermore, in the shown example, the interface is in the form of a potentiometer 60 and an associated knob 61 by means of which a user of the first bandage applicator 1 can adjust a setting of the potentiometer 60. For sake of completeness, it is noted that the first bandage applicator 1 may be equipped with suitable wiring for connecting the potentiometer 60 to the controller 51.

The controller 51 is adapted to provide an input value to the drive circuit 50, particularly an input voltage which is determined by the controller 51 in relation to the setting of the potentiometer 60. The drive circuit 50 is designed to set an input current for the motor 31 on the basis of the input voltage, and to thereby determine an output torque of the motor 31 and an associated tension of the bandage. The input voltage is received by the drive circuit 50 at a side where a operational amplifier 52 is present. The drive circuit 50 further comprises a MOSFET 53, a resistor 54, and a battery 55. The battery 55 serves as a power source, whereas the value of the current at which the motor 31 is operated is determined by the operational amplifier 52, the MOSFET 53 and the resistor 54. In particular, the MOSFET 53 is put in an opened state when the value of the current is too low, and the MOSFET 53 is put in a closed state when the value of the current is too high, wherein the operational amplifier 52 is applied for controlling the state of the MOSFET 53. The operational amplifier 52 is arranged in the circuit 50 in such a way as to be capable of serving as a voltage follower, wherein a voltage drop across the resistor 54 is always equal to the input voltage. On the basis of the well-known fact that the current is directly related to the voltage through a factor which is determined by the resistance of the resistor 54 (V = I * R, wherein V represents the voltage, I represents the current, and R represents the resistance), the input voltage directly results in a current for the motor 31, independent of a voltage prevailing across the motor 31.

On the basis of the fact that there is a direct relation between the current by means of which the motor 31 is operated and the output torque of the motor 31, it is possible to accurately control the tension prevailing in the bandage as it is unwound from the roll 40, regardless of other factors including the pulling force exerted by a user. The user can simply use the knob 61 of the potentiometer 60 for setting a desired value of the tension, as the setting of the potentiometer 60 determines the input voltage for the drive circuit 50 and the current at which the electromotor 31 is operated, resulting in a certain output torque of the motor 31 and an associated force acting on the bandage. In any practical manner, it is possible to keep track of the diameter of the bandage roll 40 in the process, in order to vary the output torque in such a manner as to keep the tension of the bandage at a certain constant level if so desired at any size of the bandage roll 40.

If different areas of a limb which is to be enwrapped in a bandage require different tension settings, a user can simply realize the correct values by putting the knob 61 of the potentiometer 60 in appropriate positions during various stages of a bandage applying process.

It follows from the foregoing that by using the first bandage applicator 1, it is possible to have accurate control of the tension in a bandage to be applied to a limb such as a leg. This means that treatments of the limb can be most effective, wherein risks that bandages are too loose or too tight are minimized, and wherein it is not necessary to rely on experience of a person who is involved in applying the bandage as far as a feeling of the correct tension in the bandage is concerned.

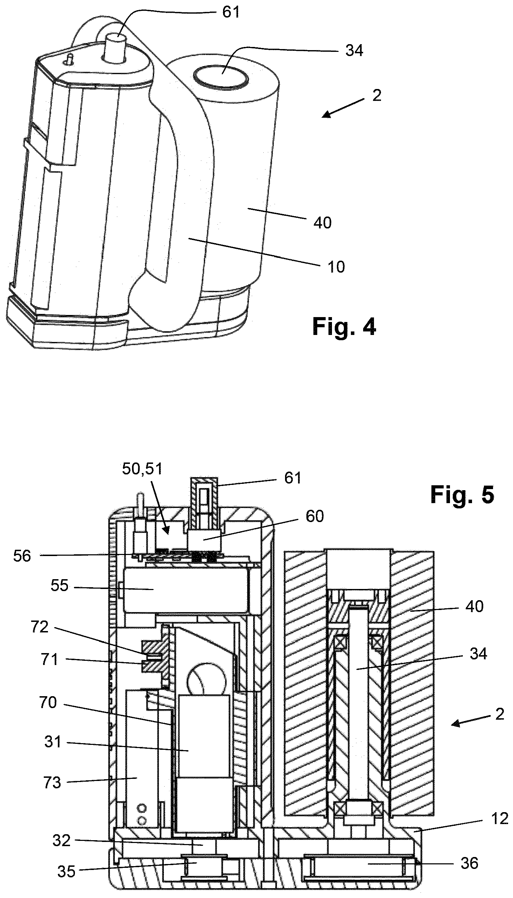

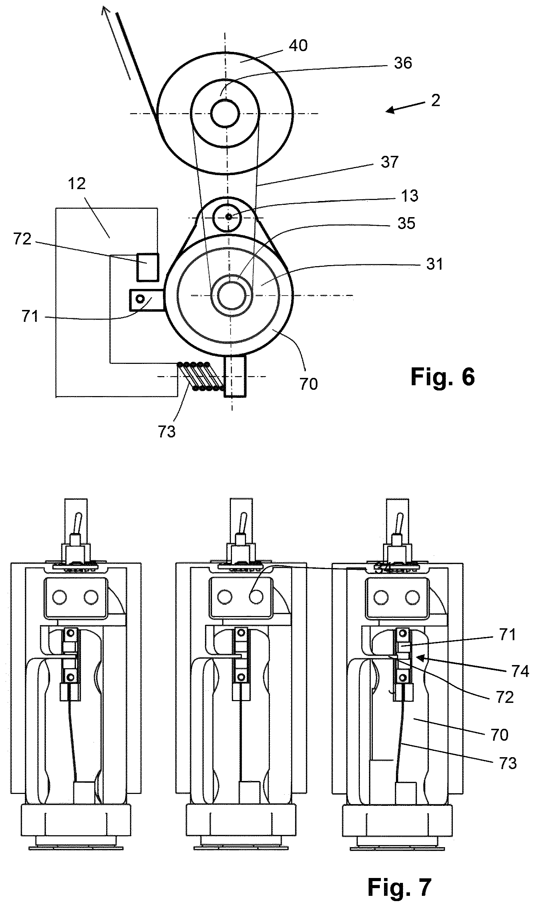

Figure 4 shows a perspective view of a second embodiment of the bandage applicator 2 according to the present invention, which will hereinafter be referred to as second bandage applicator 1, figure 5 shows a sectional view of the second bandage applicator 2, and figure 6 illustrates a mutual positioning of a number of components of the second bandage applicator, as will be explained later on. Like the first bandage applicator 1, the second bandage applicator 2 is provided in the form of a handheld device, and comprises a grip 10 for allowing a user of the device to take hold of the device. Furthermore, like the first bandage applicator 1, the second bandage applicator 2 comprises a rotatably arranged support 34 for supporting a bandage roll 40, a motor 31 for driving the support 34, a battery 55 which serves as a power source in the applicator 2, and which may be replaceable and/or rechargeable, and a potentiometer 60 and an associated knob 61 for receiving input information representing a desired tension of the bandage to be applied to a limb, among other components which are comparable to components of the first bandage applicator 1. As far as the mutual positioning of the motor 31 and the support 34 in the second bandage applicator 2 is concerned, it is noted that the support 34 is not directly connected to an output shaft 32 of the motor 31 as is the case in the first bandage applicator 1, but indirectly, through a pulley 35 connected to the output shaft 32, a pulley 36 connected to the support 34, and a toothed belt 37 arranged around both pulleys 35, 36 as mentioned. The second bandage applicator 2 comprises a support frame 12 which serves as a basic component of the applicator 2, i.e. a component on which further components of the applicator 2 are arranged, in a fixed or movable fashion, whatever is appropriate for each one of the further components. Furthermore, the second bandage applicator 2 comprises a printed circuit board 56 carrying other electrical components of a drive circuit 50 besides the battery 55, as well as electrical components of a controller 51 of the drive circuit 50.

By referring to the second bandage applicator 2, a further aspect of the way in which the operation of the motor 31 is controlled is now elucidated. The fact is that according to the present invention, the output torque of the motor 31 is not only determined on the basis of a tension of the bandage as desired, but also on the basis of feedback regarding an actual tension of the bandage during a bandage applying process. For sake of completeness, it is noted that control based on feedback as mentioned is assumed to also be applied in the first bandage applicator 1 in any suitable way, for example by using an intermediate roll for setting an appropriate force/torque, or by realizing control in a fashion which is comparable to what will now be described in respect of the second bandage applicator 2.

In the second bandage applicator 2, the motor 31 is arranged in a motor housing 70 which is rotatably arranged with respect to the support frame 12 about a rotation axis 13, as can best be seen in figure 6. At the outside of the motor housing 70, an optical sensor 71 is arranged, while the support frame 12 is equipped with a projecting element 72 which is arranged at such a position that it is possible for the optical sensor associated 71 with the motor housing 70 to detect the projecting element 72 associated with the support frame 12. Furthermore, a leaf spring 73 (which is depicted in a general sense by means of a spring symbol in figure 6) is arranged between the motor housing 70 and the support frame 12 for delimiting the extent to which the motor housing 70 is rotatable.

The second bandage applicator 2 is adapted to realize a prescribed force in the bandage during a bandage applying process. When the actual force in the bandage appears to be lower than the prescribed force, the motor 31 is controlled such as to rotate opposite to a direction in which the bandage is wound off the bandage roll 40, i.e. to pull at the bandage, so that the prescribed force can be realized after all. On the other hand, when the actual force in the bandage appears to be higher than the prescribed force, the motor 31 is controlled such as to rotate in the same direction as the direction in which the bandage is wound off the bandage roll 40, i.e. to release the bandage, so that the prescribed force can be realized after all. The moment at which the rotation of the motor 31 is reversed is determined by a precalculated deviation of the position of the leaf spring 73 with respect to a neutral position. The position of the leaf spring 73 is detected by means of the combination of the optical sensor 71 and the projecting element 72. As soon as the precalculated deviation of the position of the leaf spring 73 with respect to the neutral position is detected, a signal is transmitted to the controller 51 of the drive unit 50. The moment at which the rotation of the motor 31 is reversed can be set by means of the potentiometer 60 in view of the fact that a linear relation exists between the signal of the optical sensor 71 and a position of the projecting element 72 with respect to the optical sensor 71.

Figure 7 illustrates three possible positions of the motor housing 70 and associated positions of the leaf spring 73. In the center of figure 7, the neutral position of the leaf spring 73 is shown. At the left side of figure 7, a deviation of the position of the leaf spring 73 is shown, which is related to one direction of rotation of the motor 31, whereas at the right side of figure 7, another deviation of the position of the leaf spring 73 is shown, which is related to an opposite direction of rotation of the motor 31. It can clearly be seen that in the three positions as shown, the extent to which the projecting element 72 is present in a detection area 74 of the optical sensor 71 is different. In the shown example, the projecting element 72 is halfway the detection area 74 when the leaf spring 73 is in the neutral position. When the leaf spring 73 is in the position as shown at the left side of figure 7, the projecting element 72 covers the detection area 74 entirely, whereas when the leaf spring 73 is in the position as shown at the right side of figure 7, the projecting element 72 is just outside of the detection area 74. Hence, the exact position of the leaf spring 73 can be determined by means of the optical sensor 71. In this way, the force in the bandage can be determined, as will be explained in the following.

During operation, the motor 31 drives the bandage roll 40 by rotating the support 34. In the process, a pulling force in the bandage is transmitted as a reaction force to the motor housing 70. When the reaction force causes the motor housing 70 to rotate, the position of the leaf spring 73 is changed in view of the fact that one end of the leaf spring 73 is connected to the motor housing 70 and another end of the leaf spring 70 is connected to the support frame 12. A displacement of the end of the leaf spring 73 which is connected to the motor housing 70 is proportional to a displacement of the optical sensor 71 to the projecting element 72. As a result, the optical sensor 71 generates a signal which is proportional to the displacement of the leaf spring 73, on the basis of which it is achieved that the actual pulling force in the bandage, which acts as a reaction force on the motor housing 70, can be used as a factor in the control of the motor 31.

The desired pulling force in the bandage can be set by a user of the second bandage applicator 2 by means of the potentiometer 60. The magnitude of the pulling force as set serves as input information of the controller 51 of the drive circuit 50. The motor 31 is driven by the drive circuit 50 and generates an appropriate torque and an associated tension of the bandage. The torque of the motor 31 is transmitted to the support 34 of the bandage roll 40 through the pulleys 35, 36 and the toothed belt 37, whereas the pulling force in the bandage is transmitted to the motor housing 70 as a reaction force. As explained in the foregoing, the force is detected by means of the optical sensor 71, which provides a signal representing the force. The controller 51 is adapted to compare the value as set to the actual value, and to calculate a correction of the torque generated by the motor 31 in case a difference is found. Both an effect of the yield of the motor 31 on the torque and an effect of hysteresis in the transmission between the motor 31 and the bandage roll 40 on the torque are avoided and do not have a negative influence on control accuracy.

By using information representing the actual tension of the bandage, it is achieved that it is possible to have an immediate action of winding the bandage on the bandage roll 40 again as soon as the second bandage applicator 2 is moved towards a limb during a bandage applying process. This is advantageous in the context of applying a bandage, as in this way, loss of tension in the bandage is avoided, which would otherwise cause a part of the bandage already applied to the limb to loosen somewhat and thereby lose the ability to exert a prescribed pressure on the limb. Hence, as an advantageous result of the present invention, it is achieved that it is possible to realize the prescribed pressure on the limb under all circumstances, even when a user of the applicator 2 moves the applicator 2 in such a way which would otherwise cause the bandage to sag. On the basis of the fact that the motor 31 is controlled such as to wind the bandage on the bandage roll 40 again as soon as a force which is directed away from the limb is no longer exerted by a user, it is even possible for the user to make corrections in a part of the bandage already applied to the limb if so desired without losing pressure in a further part of the bandage which is already well in place on the limb.

It is noted that a user of the second bandage applicator 2 feels the force in the bandage during a bandage applying process. During normal operation, the user continually moves the second bandage applicator 2 around the limb to be provided with the bandage while keeping the applicator 2 at a certain distance from the limb so as to have a pulling force in the bandage, wherein the bandage is wound off the bandage roll 40. When the user determines that the force during the process of winding off is not correct, i.e. too low or too high, the user can easily adjust the setting by operating the knob 61 associated with the potentiometer 60. When the user moves the applicator 2 towards the limb, the bandage is wound on the bandage roll 40, wherein the force which is exerted on the bandage is just opposite of the force prevailing in the bandage during the process of winding off the roll 40.

It will be clear to a person skilled in the art that the scope of the present invention is not limited to the examples discussed in the foregoing, but that several amendments and modifications thereof are possible without deviating from the scope of the present invention as defined in the attached claims. While the present invention has been illustrated and described in detail in the figures and the description, such illustration and description are to be considered illustrative or exemplary only, and not restrictive. The present invention is not limited to the disclosed embodiments.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the present invention.

For sake of completeness, it is emphasized that using a rotatably arranged motor housing 70 and a leaf spring 73, wherein the leaf spring 73 is loaded by the torque acting on the motor housing 70, and wherein the extent to which a position of the leaf spring 73 diverges from a neutral position is kept constant on the basis of position control by using an optical sensor 71 which is kept at a fixed read-out, as described in respect of the second embodiment of the bandage applicator 2, is only one of a number of ways of receiving information representing an actual tension of the bandage which are possible within the framework of the present invention.

## Claims

1. Device (1, 2) for applying a bandage around a limb, the device (1, 2) being a handheld device having a grip (10) for allowing a user of the device (1, 2) to take hold of the device (1, 2), and the device (1, 2) comprising
- a rotatable support (34) for supporting a bandage roll (40);
- a motor (31) for driving the support (34);
- a bidirectional drive circuit (50) for driving the motor (31);
- an interface (60, 61) for receiving first input information representing a desired tension of the bandage during a bandage applying process;
- a detecting mechanism (70, 71, 72) for receiving second input information representing an actual tension of the bandage; and
- a controller (51) for controlling operation of the motor (31) during a bandage applying process through the drive circuit (50), which is connected to both the interface (60, 61) for receiving the first input information from the interface (60, 61) and the detecting mechanism (70, 71, 72) for receiving the second input information from the detecting mechanism (70, 71, 72), which is configured to determine a value representing an output torque of the motor (31) on the basis of the first and second input information, and which is connected to the drive circuit (50) for setting the value in the drive circuit (50).

2. Device (1, 2) according to claim 1, further comprising a support frame (12), wherein the detecting mechanism (70, 71, 72) comprises both means (70) which are movable with respect to the support frame (12) under the influence of an actual pulling force prevailing in the bandage, and means (71, 72) for detecting an actual position of the movable means (70) with respect to the support frame (12).

3. Device (1, 2) according to claim 1, further comprising a support frame (12), a motor housing (70) for accommodating the motor (31), wherein the motor housing (70) is movable with respect to the support frame (12) under the influence of an actual pulling force prevailing in the bandage, and detecting means (71, 72) for detecting an actual position of the motor housing (70) with respect to the support frame (12).

4. Device (1, 2) according to claim 3, wherein the detecting means (71, 72) comprise a combination of a projecting element (72) and an optical sensor (71) for detecting the projecting element (72), wherein the projecting element (72) is arranged on one of the support frame (12) and the motor housing (70), wherein the optical sensor (71) is arranged on another of the support frame (12) and the motor housing (70), and wherein the controller (51) is connected to the optical sensor (71).

5. Device (1, 2) according to claim 3 or 4, further comprising resilient means (73) for connecting the motor housing (70) to the support frame (12) and thereby delimiting movement of the motor housing (70) with respect to the support frame (12).

6. Device (1, 2) according to any of claims 1-5, wherein the motor (31) is an electromotor, and wherein the controller (51) is configured to determine a value representing a supply of current to the motor (31) on the basis of the first and second input information, and to control the drive circuit (50) for realizing the value.

7. Device (1, 2) according to claim 6, wherein the controller (51) is configured to determine a voltage to be set in the drive circuit (50).

8. Device (1, 2) according to claim 7, wherein the drive circuit (50) comprises a metal-oxide-semiconductor field-effect transistor (MOSFET) (53), an operational amplifier (52) and a resistor (54), wherein the operational amplifier (52) is connected to the controller (51) for receiving an incoming voltage set by the controller (51), and wherein the operational amplifier (52) is arranged for letting a voltage across the resistor (54) be the same as the incoming voltage and thereby determining a value of a current in the drive circuit (50) and determining an opened/closed state of the MOSFET (53).

9. Device (1, 2) according to any of claims 1-8, wherein the drive circuit (50) comprises a power source (55) in the form of a battery.

10. Device (1, 2) according to any of claims 1-9, wherein the interface comprises a potentiometer (60).

11. Device (1, 2) according to claims 1-10, wherein the interface is adapted to communicate with a computer through the Internet.

12. Device (1, 2) according to claim 1-11, wherein the interface is adapted to read information from a digital card.

13. Device (1, 2) according to any of claims 1-12, further comprising a memory for storing information regarding the value representing the output torque of the motor (31) as set in the drive circuit (50).

14. Device (1, 2) according to any of claims 1-13, further comprising a scanner for reading information on a bandage roll (40) representing the type of the bandage roll (40), wherein the scanner is connected to the controller (51).

## Patentansprüche

1. Vorrichtung (1, 2) zum Anbringen eines Verbands rund um eine Gliedmaße, wobei die Vorrichtung (1, 2) eine tragbare Vorrichtung ist, aufweisend einen Griff (10), um einem Benutzer der Vorrichtung (1, 2) zu ermöglichen, die Vorrichtung zu ergreifen (1, 2), und wobei die Vorrichtung umfasst:
- eine drehbare Halterung (34) zum Halten einer Verbandsrolle (40);
- einen Motor (31) zum Antreiben der Halterung (34);
- einen bidirektionalen Antriebskreis (50) zum Antreiben des Motors (31);
- eine Schnittstelle (60, 61) zum Empfangen erster Eingabeinformationen, welche eine gewünschte Spannung des Verbands während eines Verbandanbringungsvorgangs darstellen;
- einen Erfassungsmechanismus (70, 71, 72) zum Empfangen zweiter Eingabeinformationen, welche eine tatsächliche Spannung des Verbands darstellen; und
- eine Steuereinheit (51) zum Steuern des Betriebs des Motors (31) während eines Verbandanbringungsvorgangs durch den Antriebskreis (50), die sowohl mit der Schnittstelle (60, 61) zum Empfangen der ersten Eingabeinformationen von der Schnittstelle (60, 61) als auch mit dem Erfassungsmechanismus (70, 71, 72) zum Empfangen der zweiten Eingabeinformationen vom Erfassungsmechanismus (70, 71, 72) verbunden ist, und die dafür ausgelegt ist, um einen Wert zu ermitteln, der ein Ausgangsdrehmoment des Motors (31) auf der Grundlage der ersten und zweiten Eingabeinformationen darstellt, und die mit dem Antriebskreis (50) verbunden ist, um den Wert im Antriebskreis (50) einzustellen.

2. Vorrichtung (1, 2) nach Anspruch 1, ferner umfassend einen Tragrahmen (12), wobei der Erfassungsmechanismus (70, 71, 72) sowohl Mittel (70) umfasst, die in Bezug auf den Tragrahmen (12) unter dem Einfluss einer tatsächlichen, im Verband vorliegenden Zugkraft beweglich sind, als auch Mittel (71, 72) zum Erfassen einer aktuellen Position der beweglichen Mittel (70) in Bezug auf den Tragrahmen (12).

3. Vorrichtung (1, 2) nach Anspruch 1, ferner umfassend einen Tragrahmen (12), ein Motorgehäuse (70) zum Aufnehmen des Motors (31), wobei das Motorgehäuse (70) in Bezug auf den Tragrahmen (12) unter dem Einfluss einer tatsächlichen Zugkraft, die im Verband vorliegt, beweglich ist, und Erfassungsmittel (71, 72) zum Erfassen einer aktuellen Position des Motorgehäuses (70) in Bezug auf den Tragrahmen (12).

4. Vorrichtung (1, 2) nach Anspruch 3, wobei die Erfassungsmittel (71, 72) eine Kombination aus einem vorstehenden Element (72) und einem optischen Sensor (71) zum Erfassen des vorstehenden Elements (72) aufweisen, wobei das vorstehende Element (72) entweder am Tragrahmen (12) oder am Motorgehäuse (70) angeordnet ist, wobei der optische Sensor (71) am anderen der Gruppe aus Tragrahmen (12) und Motorgehäuse (70) angeordnet ist, und wobei die Steuereinheit (51) mit dem optischen Sensor (71) verbunden ist.

5. Vorrichtung (1, 2) nach Anspruch 3 oder 4, ferner umfassend elastische Mittel (73) zum Verbinden des Motorgehäuses (70) mit dem Tragrahmen (12), wodurch die Bewegung des Motorgehäuses (70) in Bezug auf den Tragrahmen (12) begrenzt ist.

6. Vorrichtung (1, 2) nach einem der Ansprüche 1-5, wobei der Motor (31) ein Elektromotor ist, und wobei die Steuereinheit (51) dafür ausgelegt ist, um einen Wert zu ermitteln, der eine Stromzufuhr zu dem Motor (31) auf der Grundlage der ersten und zweiten Eingabeinformationen darstellt, und den Antriebskreis (50) zum Umsetzen des Wertes zu steuern.

7. Vorrichtung (1, 2) nach Anspruch 6, wobei die Steuereinheit (51) dafür ausgelegt ist, um eine elektrische Spannung zu ermitteln, die im Antriebskreis (50) einzustellen ist.

8. Vorrichtung (1, 2) nach Anspruch 7, wobei der Antriebskreis (50) einen MetallOxid-Halbleiter-Feldeffekttransistor (MOSFET) (53), einen Betriebsverstärker (52) und einen Widerstand (54) umfasst, wobei der Betriebsverstärker (52) mit der Steuereinheit (51) verbunden ist, um eine durch die Steuereinheit (51) eingestellte Eingangsspannung zu empfangen, und wobei der Betriebsverstärker (52) dafür eingerichtet ist, um eine Spannung über dem Widerstand (54) gleich sein zu lassen, wie die Eingangsspannung, wodurch ein Wert eines Stroms im Antriebskreis (50) und ein geöffneter/geschlossener Zustand des MOSFET (53) ermittelt wird.

9. Vorrichtung (1, 2) nach einem der Ansprüche 1-8, wobei der Antriebskreis (50) eine Stromquelle (55) in der Form einer Batterie umfasst.

10. Vorrichtung (1, 2) nach einem der Ansprüche 1-9, wobei die Schnittstelle ein Potentiometer (60) umfasst.

11. Vorrichtung (1, 2) nach den Ansprüchen 1-10, wobei die Schnittstelle dafür ausgelegt ist, um über das Internet mit einem Computer zu kommunizieren.

12. Vorrichtung (1, 2) nach den Ansprüchen 1-11, wobei die Schnittstelle dafür ausgelegt ist, um Informationen von einer digitalen Karte auszulesen.

13. Vorrichtung (1, 2) nach einem der Ansprüche 1-12, ferner umfassend einen Speicher zum Speichern von Informationen in Zusammenhang mit dem Wert, der das Ausgangsdrehmoment des Motors (31) darstellt, das im Antriebskreis (50) eingestellt ist.

14. Vorrichtung (1, 2) nach einem der Ansprüche 1-13, ferner umfassend einen Scanner zum Ablesen von Informationen auf einer Verbandsrolle (40), welche die Art der Verbandsrolle (40) darstellen, wobei der Scanner mit der Steuereinheit (51) verbunden ist.

## Revendications

1. Dispositif (1, 2) pour application d'un bandage autour d'un membre, le dispositif (1,2) étant un dispositif manuel ayant une poignée (10) pour permettre à un utilisateur du dispositif (1, 2) de saisir le dispositif (1, 2), et le dispositif (1, 2) comprenant:
- un support rotatif (34) destiné à supporter un rouleau de bandage (40);
- un moteur (31) pour entraîner le support (34);
- un circuit d'entraînement bidirectionnel (50) pour entraîner le moteur (31);
- une interface (60, 61) pour recevoir une première information d'entrée représentant une tension voulue du bandage au cours d'une opération d'application du bandage;
- un mécanisme de détection (70, 71, 72) pour recevoir une seconde information d'entrée représentant une tension réelle du bandage; et
- un dispositif de commande (51) destiné à commander le fonctionnement du moteur (31) au cours d'une opération d'application du bandage par le biais du circuit d'entraînement (50), qui est relié à la fois à l'interface (60, 61) afin de recevoir la première information d'entrée venant de l'interface (60, 61) et au mécanisme de détection (70, 71, 72) afin de recevoir la seconde information d'entrée venant du mécanisme de détection (70, 71, 72), qui est conçu pour déterminer une valeur représentant un couple de sortie du moteur (31) sur la base de la première et de la seconde information d'entrée, et qui est relié au circuit d'entraînement (50) pour régler la valeur dans le circuit d'entraînement (50).

2. Dispositif (1, 2) selon la revendication 1, comprenant en outre une structure de support (12), où le mécanisme de détection (70, 71, 72) comprend à la fois des moyens (70) qui sont mobiles par rapport à la structure de support (12) sous l'effet d'une force de traction réelle dans le bandage, et des moyens (71, 72) pour détecter une position réelle des moyens mobiles (70) par rapport à la structure de support (12).

3. Dispositif (1, 2) selon la revendication 1, comprenant en outre une structure de support (12), un boîtier de moteur (70) pour loger le moteur (31), où le boîtier de moteur (70) est mobile par rapport à la structure de support (12) sous l'effet d'une force de traction réelle dans le bandage, et des moyens de détection (71, 72) pour détecter une position réelle du boîtier de moteur (70) par rapport à la structure de support (12).

4. Dispositif (1, 2) selon la revendication 3, dans lequel les moyens de détection (71, 72) comprennent une combinaison d'un élément saillant (72) et d'un capteur optique (71) pour détecter l'élément saillant (72), où l'élément saillant (72) est disposé sur l'un de la structure de support (12) et du boîtier de moteur (70), où le capteur optique (71) est disposé sur un autre de la structure de support (12) et du boîtier de moteur (70), et où le dispositif de commande (51) est relié au capteur optique (71).

5. Dispositif (1, 2) selon la revendication 3 ou 4, comprenant en outre des moyens élastiques (73) pour relier le boîtier de moteur (70) à la structure de support (12) et pour ainsi délimiter le mouvement du boîtier de moteur (70) par rapport à la structure de support (12).

6. Dispositif (1, 2) selon l'une quelconque des revendications 1 à 5, dans lequel le moteur (31) est un moteur électrique, et dans lequel le dispositif de commande (51) est conçu pour déterminer une valeur représentant une alimentation du moteur (31) en courant sur la base de la première et de la seconde information d'entrée, et pour commander le circuit d'entraînement (50) afin d'obtenir la valeur.

7. Dispositif (1, 2) selon la revendication 6, dans lequel le dispositif de commande (51) est conçu pour déterminer une tension à régler dans le circuit d'entraînement (50).

8. Dispositif (1, 2) selon la revendication 7, dans lequel le circuit d'entraînement (50) comprend un transistor métal-oxyde-semiconducteur à effet de champ (transistor MOS) (53), un amplificateur opérationnel (52) et une résistance (54), dans lequel l'amplificateur opérationnel (52) est relié au dispositif de commande (51) pour recevoir une tension d'entrée réglée par le dispositif de commande (51), et dans lequel l'amplificateur opérationnel (52) est prévu pour laisser une tension aux bornes de la résistance identique à la tension d'entrée et pour ainsi déterminer une valeur d'un courant dans le circuit d'entraînement (50) et déterminer un état ouvert/fermé du transistor MOS (53).

9. Dispositif (1, 2) selon l'une quelconque des revendications 1 à 8, dans lequel le circuit d'entraînement (50) comprend une source d'alimentation (55) sous la forme d'une batterie.

10. Dispositif (1, 2) selon l'une quelconque des revendications 1 à 9, dans lequel l'interface comprend un potentiomètre (60).

11. Dispositif (1, 2) selon les revendications 1 à 10, dans lequel l'interface est adaptée pour communiquer avec un ordinateur par l'Internet.

12. Dispositif (1, 2) selon les revendications 1 à 11, dans lequel l'interface est adaptée pour lire une information d'une carte numérique.

13. Dispositif (1, 2) selon l'une quelconque des revendications 1 à 12, comprenant en outre une mémoire pour mémoriser une information concernant la valeur représentant le couple de sortie du moteur (31) tel que réglé dans le circuit d'entraînement (50).

14. Dispositif (1, 2) selon l'une quelconque des revendications 1 à 13, comprenant en outre un dispositif à balayage pour lire une information sur un rouleau de bandage (40) représentant le type du rouleau de bandage (40), où le dispositif à balayage est relié au dispositif de commande (51).
